(19) Europäisches Patentamt
European Patent Office
Office européen des brevets

(11) Veröffentlichungsnummer: **0 270 915 B1**

(12) # EUROPÄISCHE PATENTSCHRIFT

(45) Veröffentlichungstag der Patentschrift: **15.07.92**

(51) Int. Cl.$^5$: **C08F 265/06**, A61K 6/08

(21) Anmeldenummer: **87117336.5**

(22) Anmeldetag: **25.11.87**

(54) **Polymerisierbare Massen, Verfahren zu ihrer Herstellung und ihre Verwendung als Dentalmassen.**

(30) Priorität: **10.12.86 DE 3642212**

(43) Veröffentlichungstag der Anmeldung:
**15.06.88 Patentblatt 88/24**

(45) Bekanntmachung des Hinweises auf die Patenterteilung:
**15.07.92 Patentblatt 92/29**

(84) Benannte Vertragsstaaten:
**AT CH DE FR GB IT LI**

(56) Entgegenhaltungen:
**EP-A- 0 011 734**
**EP-A- 0 011 761**
**WO-A-82/02556**

(73) Patentinhaber: **THERA Patent GmbH & Co. KG**
**Gesellschaft für industrielle Schutzrechte**
**Griesberg 2**
**W-8031 Seefeld 1(DE)**

(72) Erfinder: **Lechner, Günther, Dr.**
**Hurtenstrasse 8**
**W-8138 Frieding(DE)**
Erfinder: **Ellrich, Klaus, Dr.**
**Auinger Strasse 16**
**W-8031 Wörthsee(DE)**
Erfinder: **Guggenberger, Rainer, Dr.**
**Inninger Strasse 13 a**
**W-8031 Seefeld 2(DE)**
Erfinder: **Gasser, Oswald, Dr.**
**Höhenstrasse 10**
**W-8031 Seefeld 1(DE)**

(74) Vertreter: **Müller, Bernhard, Dr.**
**Graf-Toerring-Strasse 45**
**W-8031 Seefeld 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft polymerisierbare Massen, Verfahren zu ihrer Herstellung und die Verwendung dieser Massen als Dentalmassen.

Für dentale Werkstoffe, wie z. B. Zahnfüllmassen, Prothesenmaterialien, Verblendmaterialien, kieferorthopädische Basismaterialien ist es seit langem bekannt, sie aus polymerisierbaren, ethylenisch ungesättigten Monomeren (meist mono-oder polyfunktionelle Methacrylester) aufzubauen und diese mit Füllstoffen zu versetzen, um ihnen wesentliche gewünschte Eigenschaften zu verleihen. Dazu gehören: das Erzielen einer pastösen Konsistenz, die Einstellung optischer und kosmetischer Effekte, die Verringerung des Polymerisationsschrumpfs und - soweit verstärkende Füllstoffe eingesetzt werden - die Verbesserung der mechanischen Eigenschaften wie Druck- und Biegefestigkeit, sowie Oberflächenhärte.

Neben anorganischen Füllstoffen - wie Quarz oder Gläser in fein vermahlener Form oder disperse Kieselsäuren - werden auch organische Füllstoffe eingesetzt. Organische Füllstoffe sind besonders dann angezeigt, wenn spezielle Verarbeitungseigenschaften oder bestimmte optische Effekte (z. B. hohe Transparenz) oder die vollständige Verbrennbarkeit des Materials von Bedeutung sind. Wie z.B. aus "Skinner's Science of Dental Materials", W.B. Saunders Company 1982, S.177 ff, S.245 zu entnehmen ist, sind perlförmige Polymerisate aus Methylmethacrylat (ggf. mit geringfügigen Zusätzen bifunktioneller, vernetzender Monomerer), die eine mittlere Korngröße von 20 - 30 µm aufweisen, ein weit verbreiteter organischer Füllstoff. Diese Perlen werden z. B. mit Methylmethacrylat angemischt, wobei das Gemisch innerhalb weniger Minuten zu einer teigigen Masse aufquillt, das - mit den üblichen Polymerisationsinitiatoren versetzt - in geeigneten Hohlformen geformt und anschließend polymerisiert werden kann. Aufgrund des Quellverhaltens ist es allerdings nicht möglich, auf dieser Basis Ein-Komponenten-Materialien herzustellen. Die genannten Systeme werden u. a. für Prothesenkunststoffe, Verblendmaterialien, Modellierkunststoffe und Zahnfüllmaterialien eingesetzt.

Aus der DE-A 28 50 916 sind hochvernetzte Perlpolymerisate bekannt, welche nach Anmischen mit den Monomeren kaum quellen. Durch die höhere Dichte gegenüber dem Monomeranteil haben sie jedoch eine Neigung, in den Gemischen abzusetzen, so daß in der Regel anorganische Antisedimentationsmittel zur Unterdrückung dieser Tendenz eingesetzt werden müssen. Dies hat aber entscheidenden Einfluß auf die optischen Eigenschaften und die vollständige Verbrennbarkeit dieser Materialien. Außerdem wirken diese Polymerisate in der Regel nicht verstärkend.

In Houben-Weyl, Band XIV/1, S. 133ff. ist die Herstellung von voluminösen Pulvern durch Fällungspolymerisation aus Acrylsäureestern beschrieben. Spezielle derartige Polymerisate hat A. B. Wojcik in Angew. Makromol. Chem. 119, 193 (1983) und 121, 89 (1984) beschrieben. Der Einsatz derartiger Polymerisate als Füllstoff in Dentalmaterialien ist jedoch nirgends auch nur angedeutet. Daß die Verwendung derartiger Polymerisate als Füllstoffe in Dentalmaterialien gemäß dieser Literaturstelle nicht in Betracht kommt, ergibt sich nicht zuletzt aus Houben-Weyl, a.a.O., S.146, wo ausgeführt wird: "Klumpige Fällung bzw. Koagulatbildung ist dabei eine oft anzutreffende, unerfreuliche Begleiterscheinung, die durch Variation der Löslichkeitsverhältnisse überwunden werden muß."

Aufgabe der Erfindung war es, einen organischen Füllstoff für (Meth)acrylester enthaltende Massen, insbesondere Dentalmaterialien, bereitzustellen, der praktisch kein Quellverhalten aufweist, verstärkende Wirkung zeigt und stabile, nicht absetzende Ein-Komponenten-Präparate herzustellen ermöglicht. Dabei sollten die für Dentalmaterialien erforderlichen optischen, mechanischen und toxikologischen Eigenschaften erhalten bleiben.

Gegenstand der Erfindung sind somit polymerisierbare Massen auf der Basis von (Meth)-acrylsäureestern, die als Füllstoff ein Fällungspolymerisat in Form unregelmässiger Agglomeratteilchen, gebildet aus Acrylsäure- und/oder Methacrylsäureestern und gegebenenfalls anderen copolymerisierbaren Monomeren enthalten, wobei 10 bis 100 Mol-% der (Meth)acrylsäureester bi- oder polyfunktionell sind.

Es wurde erfindungsgemäß festgestellt, daß derartige Fällungspolymerisate als verstärkende Füllstoffe in Dentalmaterialien auf der Basis von mono-, bi- oder polyfunktionellen (Meth)acrylsäureestern eingesetzt werden können. Diese Füllstoffe ermöglichen die Herstellung von Ein-Komponenten-Präparaten, da sie weder Quell- noch Absetzverhalten aufweisen. Außerdem ermöglichen sie die Herstellung rückstandsfrei verbrennbarer Massen.

Die Fällungspolymerisate bestehen, wie vorstehend erwähnt, aus Acrylsäure- und/oder Methacrylsäureestern; ein Zusatz anderer copolymerisierender Monomerer ist möglich, soweit die Eigenschaften der Fällungspolymerisate nicht negativ beeinträchtigt werden. Beispiele für derartige Comonomere sind Styrol, Vinylether, ungesättigte Carbonsäuren und deren Ester, Fumarsäureester, Maleinsäureester, Acrylnitril und dergl. Mindestens 10 Mol-% der (Meth)acrylsäureester müssen bi- oder polyfunktionell sein; vorteilhaft ist ein Anteil dieser Ester von mindestens 20 Mol.-%, bevorzugt sind mindestens 60 Mol.-% und besonders

geeignete Fällungspolymerisate werden zu 100 % aus bi- oder polyfunktionellen (Meth)acrylsäureestern gebildet.

Geeignete bifunktionelle (Meth)acrylester sind Bis-GMA (Reaktionsprodukt aus Bisphenol A mit 2 Moläquivalent Glycidyl-methacrylat), Triethylenglykoldimethacrylat, Ethylenglykoldimethacrylat und die in der DE-C 19 21 869, DE-C 28 16 823 und DE-A 36 07 331 genannten (Meth)acrylsäureester.

Die erfindungsgemäßen Fällungspolymerisate bestehen aus unregelmäßigen Agglomeratpartikeln, die eine Korngröße von etwa 0,2 bis 200 µm aufweisen. Das Gewichtsmittel der Kornverteilung der Sekundäraggregate liegt zwischen 5 µm and 100 µm, bevorzugt sind Fällungspolymerisate mit einem Gewichtsmittel der Korngröße zwischen 10 µm and 50 µm. In geringem Umfang können auch nicht-agglomerierte Primärteilchen vorliegen.

Die Agglomerate werden aus Primärteilchen gebildet, deren Korngröße zwischen 0,05 µm and 2 µm liegt. Bevorzugt sind Fällungspolymerisate, deren Primärteilchen eine Korngröße zwischen 0,1 µm and 1 µm aufweisen.

Die Korngröße der Sekundärteilchen kann z. B. mittels Siebanalyse oder besonders vorteilhaft mit der Laser-Granulometrie ermittelt werden. Die Größe der Primärteilchen ergibt sich aus der Vermessung von rasterelektronenmikroskopischen Aufnahmen; geeigneterweise werden dabei Teilchen vermessen, die an der Peripherie der Agglomerate sitzen und nur soweit zu dem Agglomerat verschmolzen sind, daß ihr Durchmesser erkennbar ist.

Ein weiteres Kennzeichen der erfindungsgemäßen Fällungspolymerisate ist ihre BET-Oberfläche: Diese liegt im Bereich zwischen 5 und 300 $m^2/g$; bevorzugt sind Polymerisate mit einer Oberfläche zwischen 10 und 200 $m^2/g$, insbesondere sind Polymerisate mit einer Oberfläche zwischen 50 und 100 $m^2/g$ geeignet.

Nachstehend wir die Herstellung der Fällungspolymerisate näher beschrieben.

Vorteilhafterweise erfolgt die Herstellung der Fällungspolymerisate in Analogie zum Verfahren der Dispersionspolymerisation. Die Herstellung wird beschrieben von K. E. J. Barrett, Brit. Polym. J. 5, 259 - 271 (1973) und K. Almog et al, Brit. Polym. J. 14, 131 (1982), sowie in den nachstehenden Herstellungsbeispielen.

Obwohl nach der Vorschrift einer Dispersionspolymerisation vorgegangen wird, liegt in Wirklichkeit eine Fällungspolymerisation vor, da die entstandenen Polymerisate durch ihren hohen Anteil an bi- und polyfunktionellen (Meth-)acrylsäureestern so schwer löslich sind, daß sie auch durch den zugegebenen Dispersionsstabilisator nicht in Schwebe gehalten werden können. Durch das Zusammenlagern der ausfallenden Polymerteilchen entstehen daher keine Perlen, wie bei der Dispersionspolymerisation, sondern poröse Agglomerate, bestehend aus kugeligen Primär- und unregelmäßigen Sekundärteilchen.

Prinzipiell ist die Herstellung der erfindungsgemäßen Polymerisate auch nach den Verfahren der Fällungspolymerisation möglich, wie sie z. B. in Houben-Weyl, Band XIV/1 beschrieben wird.

Nachstehend wir die Verwendung der Fällungspolymerisate näher erläutert.

Die Fällungspolymerisate werden als verstärkende Füllstoffe in Dentalmaterialien auf der Basis von (Meth-)acrylsäureestern eingesetzt, wobei sie geringe Neigung zur Quellung und zum Absetzen zeigen. Die Fällungspolymerisate werden dabei in Gewichtsanteilen von 2 - 60 Gew.-%, bevorzugt von 5 - 40 Gew.-% und besonders bevorzugt von 5 - 25 Gew.-%, jeweils bezogen auf die Gesamtmasse, eingesetzt. Sie können zusammen mit anderen anorganischen und organischen Füllstoffen verwendet werden.

Geeignete Monomere für die erfindungsgemäßen Dentalmaterialien sind mono-, bi- oder polyfunktionelle (Meth)acrylsäureester. Besonders geeignete bifunktionelle (Meth)acrylsäureester sind Bis-GMA, Triethylenglykoldimethacrylat und die in DE-C 19 21 869, DE-C 28 16 823 und DE-A 36 07 331 genannten (Meth)-acrylsäureester.

Bei den (Meth)acrylsäureestern der DE-A 36 07 331 handelt es sich um Produkte der allgemeinen Formel

$$(MO)_n\left[A-O-CO-CH_2-O-CH_2-CH_2-O-CH_2-CO-O\right]_x A-(OM)_n$$

worin bedeuten:

A einen Alkoholrest mit mindestens 4 Kettenatomen zwischen zwei Verknüpfungsstellen

M $CH_2=C(R^1)-CO-$,
wobei $R^1$ Wasserstoff oder Methyl bedeutet

n 1 oder 2,

x 0,3 bis 3.

Die erfindungsgemäßen Dentalmaterialien können neben den erfindungsgemäßen Fällungspolymerisaten und den Monomeren die dem Fachmann bekannten Polymerisationsinitiatoren enthalten:

Durch Zusatz von Peroxiden, wie Benzoylperoxid oder Lauroylperoxid, AIBN oder anderen thermischen Radikalbildnern entstehen Massen, die nach in der Dentaltechnik bekannten Verfahren bei erhöhter Temperatur, ggf. unter Druck, ausgehärtet werden.

Zur Kalthärtung können z. B. die Initiator-Systeme Peroxid/Amin, Hydroperoxid/Thioharnstoffderivat, Barbitursäure/Peroxid/$Cu^{2+}$-Ionen/$Cl^{-}$-Ionen eingesetzt werden. Dabei werden die Initiator-Bestandteile auf unterschiedliche Komponenten der erfindungsgemäßen Dentalmasse verteilt.

Besonders geeignet sind die erfindungsgemäßen Fällungspolymerisate zur Herstellung von lediglich aus einer Komponente bestehenden, photopolymerisierbaren Dentalmassen. Dabei werden den Massen 0,1 bis 10 Gew.-%, bezogen auf den Monomeranteil, an bekannten Photoinitiatoren zugesetzt. Die Polymerisationsauslösung erfolgt entweder mit UV-Licht oder, in bevorzugter Weise, mit sichtbarem Licht des Wellenlängenbereichs von 400 - 550 nm. Geeignete Photoinitiatoren sind Benzophenon, Benzilketale, Benzoinether und - insbesondere für die Polymerisation mit sichtbarem Licht - aromatische oder aliphatische Diketone wie Benzil, Phenanthrenchinon oder Campherchinon sowie Mono-oder Bisacylphosphinoxide.

Darüber hinaus können die Massen die üblichen Stabilisatoren, Inhibitoren, Farbstoffe, Pigmente, Trübungsmittel und/oder Röntgenkontrastmittel enthalten.

Die erfindungsgemäßen Fällungspolymerisate finden zur Herstellung von Zahnfüllmassen, Prothesenmaterialien, Verblendwerkstoffen, Modellierkunststoffen, kieferorthopädischen Materialien und ähnlichem Verwendung. Ihre Verwendung ist besonders vorteilhaft, wenn die rückstandsfreie Verbrennung des ausgehärteten Materials, wie z. B. einem Modellierwerkstoff, erforderlich ist. Eine derartige Anwendung ist z. B. in der DE-A 32 40 907 beschrieben. Aber auch zur Herstellung von Materialien mit hoher Transparenz, wie z. B. kieferorthopädischen Materialien, können die erfindungsgemäßen Fällungspolymerisate besonders vorteilhaft eingesetzt werden. Die Eigenschaften derartiger Materialien sind den Beispielen zu entnehmen.

Nachstehend wir die Erfindung anhand von Beispielen näher erläutert.

Beispiel 1

Herstellung eines Fällungspolymerisats

In 400 ml Methanol werden bei Raumtemperatur gelöst:

18 g Methylmethacrylat
12 g Glykoldimethacrylat
0,3 g Azoisobuttersäurenitril
7 g Polyvinylpyrrolidon

Das Reaktionsgemisch wird 24 h bei 60 °C gerührt. Der entstandene Niederschlag wird abgesaugt und getrocknet. Das Produkt hat eine BET-Oberfläche von 8,3 $m^2/g$ und eine mittlere Sekundärteilchengröße von 31,6 µm.

Die durch Lasergranulometric ermittelte Korngrößenverteilung der Sekundärteilchen ist in Tabelle I angegeben.

Beispiel 2

A. Herstellung eines Methacrylsäureestergemisches (gemäß Beispiel 1 der DE-A 36 07 331)

Triglykolsäure-bis [3(4)-methacryloxymethyl-8(9)-tricyclo [$5.2.1.0^{2,6}$]decylmethylester].

196 g (1,0 Mol) Bis-(hydroxymethyl)-tricyclo[$5.2.1.0^{2,6}$]decan (T-diol), gelöst in 400 ml Cyclohexan, werden unter Ausschleppen von 18 g Wasser mit 89 g (0,5 Mol) Triglykolsäure teilverestert. Als Katalysator verwendet man 7 g p-Toluolsulfonsäure. Die restlichen Hydroxylfunktionen des Diols werden unter Zugabe von 80 mg Pikrinsäure, 200 mg Methylenblau und 35 mg BHT als Polymerisationsinhibitoren mit 129 g (1,5 Mol) frisch destillierter Methacrylsäure verestert, wobei sich weitere 18 g Wasser abscheiden. Das rohe Estergemisch verdünnt man mit weiteren 400 ml Cyclohexan und 250 ml Toluol und wäscht mit 4 x 350 ml 2-n NaOH, 2 x 350 ml 0,5- n $H_2SO_4$ sowie mit Wasser. Man trocknet die Esterlösung mit Natriumsulfat, durchperlt sie mit Luftsauerstoff und reinigt abschliessend über Aluminiumoxid. Nach Zugabe von 4-Methoxyphenol zur Inhibierung erhält man durch Konzentrieren im Hochvakuum 258 g eines Estergemisches folgender durchschnittlicher Zusammensetzung:

32 % T-diol-bis-methacrylat
38 % Triglykolsäure-bis T-methacrylat
30 % Bis-(methacrylat) von

$$OH-\left[T-O-CO-CH_2-O-CH_2-CH_2-O-CH_2-CO-O\right]_{2-3}-T-OH$$

Viskosität: 80 dPa.s
Dichte: 1,152 g/ml
$n_P^{20}$ : 1,496

Das Methacrylsäureestergemisch entspricht der vorstehend unter Hinweis auf die DE-A 36 07 331 angegebenen allgemeinen Formel mit folgenden Bedeutungen:
A = Bis-(hydroxymethyl)-tricyclo[5.2.1.$0^{2,6}$]decan
M = $CH_2 = C(CH_3)$-CO-
n = 1
x = 1,3.

B. Herstellung des Fällungspolymerisats

Die Herstellung erfolgt analog Beispiel 1, jedoch aus:
15 g Methacrylsäureestergemisch gemäss Abschnitt A.
9 g Polyglykol-400-dimethacrylat
6 g Bishydroxymethyl-bicyclo[5.2.1.$0^{2,6}$]decandiacrylat
7 g Polyvinylpyrrolidon
0,3 g Azoisobuttersäurenitril

Das getrocknete Produkt hat eine BET-Oberfläche von 15,9 $m^2$/g und eine mittlere Sekundärteilchengröße von 34,3 µm.

Die durch Lasergranulometrie ermittelte Korngrößenverteilung der Sekundärteilchen ist in Tabelle I angegeben.

Beispiel 3

Die Herstellung erfolgt analog Beispiel 1, jedoch aus:
15 g Glykoldimethacrylat
15 g Bishydroxymethyl-bicyclo[5.2.1.$0^{2,6}$]decandiacrylat
7 g Polyvinylpyrrolidon
0,3 g Azoisobuttersäurenitril

Das getrocknete Produkt hat eine BET-Oberfläche von 72,3 $m^2$/g und eine mittlere Sekundärteilchengröße von 16,2 µm.

Die durch Lasergranulometrie ermittelte Korngrößenverteilung der Sekundärteilchen ist in Tabelle I angegeben.

Tabelle I

| Beispiel 1 | | Beispiel 2 | | Beispiel 3 | |
|---|---|---|---|---|---|
| µm | Verteilung | µm | Verteilung | µm | Verteilung |
| 1 | 002,2 % | 1 | 001,5 % | 1 | 001,6 % |
| 1,5 | 002,5 % | 1,5 | 001,7 % | 1,5 | 001,9 % |
| 2 | 003,1 % | 2 | 002,4 % | 2 | 002,9 % |
| 3 | 003,9 % | 3 | 003,6 % | 3 | 004,6 % |
| 4 | 005,4 % | 4 | 005,4 % | 4 | 006,9 % |
| 6 | 007,4 % | 6 | 007,8 % | 6 | 012,3 % |
| 8 | 010,2 % | 8 | 010,9 % | 8 | 019,3 % |
| 12 | 015,5 % | 12 | 016,0 % | 12 | 034,6 % |
| 16 | 022,7 % | 16 | 022,8 % | 16 | 049,3 % |
| 24 | 035,7 % | 24 | 032,3 % | 24 | 072,1 % |
| 32 | 050,8 % | 32 | 046,1 % | 32 | 086,2 % |
| 48 | 076,1 % | 48 | 072,8 % | 48 | 098,5 % |
| 64 | 086,9 % | 64 | 085,5 % | 64 | 098,7 % |
| 96 | 099,8 % | 96 | 100,0 % | 96 | 099,9 % |
| 128 | 099,9 % | 128 | 100,0 % | 128 | 099,9 % |
| 192 | 100,0 % | 192 | 100,0 % | 192 | 100,0 % |

50 %-Punkt = 031,6    50 %-Punkt = 034,3    50 %-Punkt = 016,2

Beispiel 4

Photopolymerisierbare Masse zur Herstellung von kieferorthopädischen Apparaten.

Es werden innig vermischt:
41 g Methacrylsäureestergemisch gemäß Beispiel 2A
25,5 g Polyglykol-400-dimethacrylat
17 g Bishydroxymethyl-bicyclo [5.2.1.$0^{2,6}$]decandiacrylat
15,2 g Polymerisat laut Beispiel 2
0,3 g Campherchinon
1 g Methyldiethanolamin

Die gut verstreichbare Masse wird durch Bestrahlen mit Licht mit einer Wellenlänge zwischen 400 und 500 nm erhärtet. Ein praktisch schmierschichtfreies und transparentes Formteil wird erhalten durch Polymerisation im Vakuum mit dem Lichtpolymerisationsgerät Visio-Beta der Fa. ESPE. Auch bei längerem Lagern bleibt die Masse praktisch unverändert.

Polymerisierbare Schichtdicke: 12 mm
Druckfestigkeit: 412 MPa

Beispiel 5

Photopolymerisierbarer Dentalwerkstoff, rückstandsfrei verbrennbar, zur Herstellung von individuellen Gußteilen in der Zahntechnik (vgl. DE-A 32 40 907).

Man verarbeitet zu einem fließfähigen Gemisch:
54 g Methacrylsäureestergemisch gemäß Beispiel 2A
37 g Diacrylat von ethoxyliertem Bisphenol A
7,5 g Polymerisat laut Beispiel 3
0,2 g Campherchinon
1,3 g Triethanolamin
0,01 g Sudan-Blau (BASF)

Mit dieser Masse sind in einfacher Weise Gußmodelle aufzubauen. Polymerisation erfolgt durch Bestrahlen mit Licht einer Wellenlänge zwischen 400 und 500 nm z. B. mit den Polymerisationsgeräten Visio-Alfa und Elipar der Fa. ESPE. Die erhaltenen Gußmodelle verbrennen rückstandsfrei. Auch nach längerer Lagerzeit ist keine Viskositätsveränderung durch Quellen und praktisch keine Entmischung feststellbar.

| | |
|---|---|
| Polymerisierbare Schichtdicke: | 8 mm |
| Druckfestigkeit: | 380 MPa |

Beispiel 6

Photopolymerisierbares Material zur Herstellung und Reparatur von Kunststoffprothesen.

Man verarbeitet zu einer teigartigen Masse:
64,7 g Methacrylsäureestergemisch gemäß Beispiel 2A
21,5 g Bishydroxymethyl-bicyclo [5.2.1.$0^{2,6}$]decandiacrylat
12,5 g Polymerisat laut Beispiel 1
0,3 g Campherchinon
1 g Dimethylaminoethanolmethacrylat
4 mg Sico-Echtrot (BASF)

Auf einem Gipsmodell kann aus dieser Masse eine Prothese aufgebaut oder eine gebrochene Prothese repariert werden. Diese Masse ist über längere Zeit praktisch viskositäts- und entmischungsstabil.

Die Polymerisation erfolgt wie in Beispiel 4.

| | |
|---|---|
| Polymerisierbare Schichtdicke: | 6 mm |
| Druckfestigkeit: | 350 MPa |

Vergleichsbeispiel

Wird in Beispiel 6 anstelle des Fällungspolymerisates ein käufliches Perlpolymerisat von vollvernetzten Perlen eingesetzt, so ergibt sich bei vergleichbarer Pastenkonsistenz lediglich eine Druckfestigkeit von 65 MPa. Außerdem setzt sich dieser Füllstoff bereits nach wenigen Tagen ab.

Beispiel 7

Photopolymerisierbarer Dentalwerkstoff zur Herstellung von individuellen Abdrucklöffeln

Es werden vermischt:
30,0 g Bis-GMA
60,0 g Triglykoldimethacrylat
10,0 g Polymerisat gemäß Beispiel 3
0,3 g Campherchinon
1,0 g Methyldiethanolamin

Es wird eine entmischungsstabile Masse erhalten, die auf einem Gipsmodell zu einem individuellen Abdrucklöffel verarbeitet werden kann.

Die Polymerisation erfolgt wie unter Beispiel 4 beschrieben.

| | |
|---|---|
| Schichtdicke: | 8 mm |
| Druckfestigkeit: | 282 MPa |

Beispiel 8:

Photopolymerisierender Dentalwerkstoff, rückstandsfrei verbrennbar für Modellierzwecke in der Zahntechnik

Eine Paste wird hergestellt aus:
30,00 g Bis-GMA
60,00 g Triglykoldimethacrylat
8,50 g Polymerisat gemäß Beispiel 1
0,30 g Campherchinon
1,00 g Methyldiethanolamin
0,01 g Sudanblau (BASF)

Das erhaltene Modelliermaterial ist entmischungsstabil und rückstandsfrei verbrennbar.

Die Verwendung und Polymerisation erfolgt wie unter Beispiel 5 beschrieben.

| | |
|---|---|
| Schichtdicke: | 7 mm |
| Druckfestigkeit: | 253 MPa |

## Patentansprüche

1. Polymerisierbare Massen auf der Basis von (Meth)acrylsäureestern, enthaltend als Füllstoff ein Fällungspolymerisat in Form unregelmässiger Agglomeratteilchen, gebildet aus Acrylsäure- und/oder Methacrylsäureestern und gegebenenfalls anderen copolymerisierbaren Monomeren, wobei 10 bis 100 Mol-% der (Meth)acrylsäureester bi- oder polyfunktionell sind.

2. Massen nach Anspruch 1, dadurch gekennzeichnet, daß der Anteil der Fällungspolymerisate in der Gesamtmasse 2 bis 60 Gew.-% und insbesondere 5 bis 40 Gew.-% beträgt.

3. Massen nach Anspruch 1, dadurch gekennzeichnet, daß die Fällungspolymerisate folgende Eigenschaften aufweisen:
   a) sie bestehen aus im wesentlichen unregelmäßigen Agglomeratteilchen (Sekundärteilchen) mit einer Korngröße von 0,2 bis 200µm,
   b) das Gewichtsmittel der Korngrösse der Sekundärteilchen beträgt 5 bis 100µm,
   c) die Sekundärteilchen sind aus Primärteilchen gebildet, deren Korngröße 0,05 bis 2µm beträgt und
   d) die BET-Oberfläche beträgt 5 bis 300 $m^2$/g.

4. Massen nach Anspruch 3, dadurch gekennzeichent, daß die Fällungspolymerisate folgende Eigenschaften aufweisen:
   e) das Gewichtsmittel der Korngröße der Sekundärteilchen beträgt 10 bis 50 µm,
   f) die Korngröße der Primärteilchen beträgt 0,1 bis 1µm und
   g) die BET-Oberfläche beträgt 10 bis 200 und insbesondere 50 bis 100 $m^2$/g.

5. Verfahren zur Herstellung der Massen nach A 1-4, dadurch gekennzeichnet, daß man (Meth)-acrylsäureester, Fällungspolymersiate gemäß Anspruch 1-4 und gegebenenfalls Initiatoren, Stabilisatoren, Inhibitoren, Farbstoffe, Pigmente, Trübungsmittel und/oder Röntgenkontrastmittel vermischt.

6. Verwendung der Massen nach Anspruch 1-4 als Dentalmassen.

## Claims

1. Polymerizable compositions on the basis of (meth)acrylic acid esters containing as filler a precipitation polymer in the form of irregular agglomerate particles formed from acrylic acid and/or methacrylic acid esters and possibly other copolymerizable monomers, 10 to 100 mol% of the meth(acrylic acid esters being bi or polyfunctional.

2. Compositions according to claim 1, characterized in that the proportion of the precipitation polymers in the total composition is 2 to 60% by weight and in particular 5 to 40% by weight.

3. Compositions according to claim 1, characterized in that the precipitation polymers have the following

properties:
a) they consist of substantially irregular agglomerate particles (secondary particles) having a grain size of 0.2 to 200 µm,
b) the weight average of the grain size of the secondary particles is 5 to 100 µm,
c) the secondary particles are formed from primary particles having a grain size of 0.05 to 2 µm and
d) the BET surface area is 5 to 300 $m^2$/g.

**4.** Compositions according to claim 3, characterized in that the precipitation polymers have the following properties:
e) the weight average of the grain size of the secondary particles is 10 to 50 µm,
f) the grain size of the primary particles is 0.1 to 1 µm and
g) the BET surface area is 10 to 200 and in particular 50 to 100 $m^2$/g.

**5.** Process for the preparation of the compositions according to claims 1 to 4, characterized in that (meth)-acrylic acid esters, precipitation polymers according to claims 1 to 4 and possibly initiators, stabilizers, inhibitors, colouring agents, pigments, opacifying agents and/or X-ray contrast agents are mixed together.

**6.** Use of the compositions according to claims 1 to 4 as dental compositions.

**Revendications**

**1.** Masses ou pâtes polymérisables à base d'esters d'acide (méth)acrylique contenant en tant que matière de charge un polymérisat de précipitation sous forme de particules d'agglomérat irrégulières, formées à partir d'esters de l'acide acrylique et/ou de l'acide méthacrylique et éventuellement d'autres monomères copolymérisables, 10 à 100 moles % des esters de l'acide (méth)acrylique étant bi- ou polyfonctionnels.

**2.** Masses ou pâtes selon la revendication 1, caractérisées en ce que la proportion de polymérisat de précipitation dans la masse totale est de 2 à 60 % en poids et en particulier de 5 à 40 % en poids.

**3.** Masses ou pâtes selon la revendication 1, caractérisées en ce que les polymérisats de précipitation présentent les propriétés suivantes :
a) ils sont constitués essentiellement de particules d'agglomérat irrégulières (particules secondaires) ayant une granulométrie de 0,2 jusqu'à 200 µm,
b) la moyenne pondérale des particules secondaires est de 5 à 100 µm,
c) les particules secondaires sont constituées par les particules primaires dont la granulométrie est de 0,05 jusqu'à 2 µm, et
d) la surface BET est de 5 à 300 $m^2$/g.

**4.** Les masses ou pâtes selon la revendication 3 sont caractérisées en ce que les polymérisats de précipitation présentent les propriétés suivantes :
e) la moyenne pondérale des particules secondaires est de 10 à 50 µm,
f) la granulométrie des particules primaires est de 0,1 jusqu'à 1 µm, et
g) la surface BET est de 10 à 200 et notamment de 50 à 100 $m^2$/g.

**5.** Procédé pour la préparation de masses ou pâtes selon les revendications 1-4, caractérisé en ce que l'on mélange des esters de l'acide (méth)acrylique, des polymérisats de précipitation selon les revendications 1-4 et éventuellement des amorçeurs, stabilisateurs, inhibiteurs, colorants, pigments, opacifiants et/ou agents de contraste aux rayons X.

**6.** Utilisation des masses ou pâtes selon les revendications 1-4 en tant que masses ou pâtes dentaires.